# NOUVEAU FASCICULE DE BREVET EUROPEEN

(11) **EP 0 716 849 B2**
(45) Date de publication et mention de la décision concernant l'opposition: **27.09.2000**
(45) Mention de la délivrance du brevet: 19.03.1997
(21) Numéro de dépôt: 95402559.9
(22) Date de dépôt: 15.11.1995
(51) Int. Cl.: A61K 7/48

(54) **Composition cosmétique ou dermatologique contenant un mélange de céramides, son utilisation pour hydrater la peau**
Kosmetische oder dermatologische Zusammensetzung, die eine Mischung von Ceramiden enthält, und ihre Verwendung zur Befeuchtung der Haut
Cosmetic or dermatological composition containing a mixture of ceramides and its use for moisturizing the skin

(30) Priorité: 14.12.1994 FR 9415074
(43) Date de publication de la demande: 19.06.1996
(73) Titulaire: L'OREAL, 75008 Paris (FR)
(72) Inventeur: Candau, Didier, F-91570 Bievres (FR); Khayat, Carine, F-94210 La Varenne Saint Hilaire (FR); Nadaud, Jean-François, F-92140 Clamart (FR); Agnus-Ancilotti, Dominique, F-94210 La Varenne Saint Hilaire (FR)
(74) Mandataire: Tezier Herman, Béatrice

(56) Documents cités:
- WO-A-90/01323
- WO-A-94/00127
- JP-A- 61 260 008
- US-A- 5 368 857
- EUROPEAN JOURNAL OF DERMATOLOGY, vol. 1, no. 1, Octobre 1991 MÜNCHEN (GERMANY), pages 39-43, M. ERSCHER 'Skin ceramides: Structure and function'
- PATENT ABSTRACTS OF JAPAN vol. 11 no. 115 (C-415) [2562] ,10 Avril 1987 & JP-A-61 260008 (SUNSTAR INC.) 18 Novembre 1986,
- Wertz et al. (1985) J. of Invest. Derm. 84, 410-412
- Merck-Index page 1380

## Description

L'invention se rapporte à une composition cosmétique ou dermatologique contenant un mélange de céramides et notamment un mélange de céramide de type III et d'autres types de céramides, permettant notamment d'hydrater la peau, aussi bien du visage que du corps, voire même du cuir chevelu, et des ongles. Elle se rapporte plus particulièrement à une composition hydratante de ce type.

L'invention se rapporte aussi à l'utilisation de cette composition pour hydrater la peau ou préparer une pommade ou un onguent dermatologique pour traiter la peau très sèche ainsi qu'à un procédé de traitement cosmétique de la peau. Elle concerne aussi un procédé pour abaisser le point de fusion d'un céramide de type III.

La peau du corps et plus spécialement celle du visage est constamment soumise aux agressions de l'environnement tel que le vent, le froid, la poussière, conduisant à une perte en eau importante qu'il faut sans cesse compenser. Une déshydratation de la peau se traduit par une peau souvent ridée, rêche, rugueuse, ayant tendance à se desquamer et ayant perdue de son élasticité. En outre, la déshydratation, hormis dans le cas de maladies de peau, est souvent synonyme d'une peau âgée. Or, on cherche de plus en plus à paraître jeune et moins ridé.

On trouve sur le marché de nombreuses compositions cosmétiques ou dermatologiques destinées à traiter les peaux sèches ou à tendance sèche. Pour ce faire, elles contiennent des actifs hydratants comme les polyols (glycérine) conférant, malheureusement, à ces compositions un toucher souvent collant, écartant ainsi une grande partie des consommateurs de leur utilisation. Elles peuvent aussi contenir des hydroxyacides et/ou leurs sels qui ont l'inconvénient de piquer, d'irriter et de chauffer la peau, ce qui procure un certain inconfort pour l'utilisateur. On trouve aussi des compositions qui renferment comme actifs hydratants ou empêchant la déshydratation des huiles ou autres corps gras, conduisant à des compositions souvent longues à faire pénétrer dans la peau, laissant un film gras sur la peau, ce qui est peu apprécié des utilisateurs.

Aussi, on cherche régulièrement à introduire dans les compositions cosmétiques ou dermatologiques de nouveaux actifs hydratants ou empêchant la déshydratation de la peau en restaurant son effet barrière.

Récemment, on a envisagé l'utilisation de céramides ou pseudocéramides comme agent hydratant et/ou restructurant de la peau en vue notamment de prévenir et/ou lutter contre le vieillissement cutané (voir notamment les documents EP-A-556957, EP-A-587288, EP-A-542549). Dans ce sens également, il a été décrit dans la demande de brevet WO 90/01323 une composition comprenant un mélange constitué d'acides gras libres, de stérols ou leurs esters et de phospholipides ou glycolipides. Ces trois classes de lipides présents sont des produits venant du stratum corneum de la peau. Ainsi, les céramides éventuellement compris dans ce mélange sont des céramides naturels et sont donc tous purs.

Les céramides occupent une place majeure dans la peau et surtout dans les couches supérieures de l'épiderme, c'est-à-dire dans le *stratum corneum.* Il existe plusieurs types de céramides selon leur localisation et leur fonction au sein de l'épiderme. Le terme de céramide, pris dans son sens strict, comprend uniquement les lipides constitués de la famille des sphingosines telles que la sphinganine, la 4-hydroxysphinganine, la phytosphingosine, reliée à un acide gras ou dérivé d'acide gras par sa fonction amine.

Les céramides du *stratum corneum* sont constitués de 6 fractions, chromatographiquement distinctes, ayant une polarité différente selon leur degré d'insaturation (qui peut être nul) ou d'hydroxylation de leurs chaînes, leur longueur et leur nombre. Selon leur configuration chimique, elles sont répertoriées en classe I, II, III, IV, V, Vla et Vlb. Leur configuration chimique est notamment donnée dans le document Ceramides, Key components for skin protection de R. D. Petersen, Cosmetics & Toiletries, vol. 107, février 1992, p.45-49 et le document EJD n°1, vol. 1, Octobre 1991, Review article p.39-43 Skin ceramides : structure and function de M. Kerscher.

Les lipides du ciment inter-cornéocytaire de la peau et notamment les céramides sont organisés en bi-couches lamellaires ou feuillets et participent à la cohésion du *stratum corneum* en vue de maintenir l'intégralité de la barrière et son rôle protecteur, antipénétration, anti-irritation etc.

Le plus important pour la propriété barrière est l'état physique des bi-couches, sous forme lamellaires.

Les céramides introduits dans les compositions cosmétiques ou dermatologiques peuvent être extraits de la peau ou bien synthétisés. L'extraction de la peau n'est pas toujours aisée. En outre, il n'est pas possible d'obtenir, par synthèse chimique classique, un céramide pur pouvant conduire, soit hydraté, soit associé à d'autres lipides, à une structure en feuillets, comme les céramides de la peau. On obtient toujours un mélange racémique, ce qui pose des problèmes de purification (difficulté et longueur des procédés).

Seuls les céramides de type III synthétisés à ce jour présentent l'avantage de posséder la même structure stéréolsomérique que les céramides de la peau et d'être purs. Du fait de cette stéréoïsomérie, ces céramides peuvent s'organiser en feuillets comme le ciment intercellulaire. Il s'en suit une meilleur compatibilité avec la peau et une efficacité accrue par rapport aux céramides ayant une structure différente. Cette stéréoïsomérie résulte d'une fabrication de ces céramides par voie enzymatique en utilisant des enzymes stéréospécifiques.

La demanderesse a donc envisagé l'introduction des céramides de type III, obtenus par voie enzymatique, dans des compositions cosmétiques ou dermatologiques en vue de restructurer et/ou hydrater la peau et/ou de lutter contre le vieillissement. Malheureusement, ces céramides présentent un point de fusion élevé, de l'ordre de 126 °C, incompatible avec les ingrédients classiquement utilisés dans les domaines considérés. Or, pour assurer leur dissolution dans les milieux cosmétiques ou dermatologiques, il est nécessaire de les porter à leur température de fusion risquant ainsi de détériorer les autres constituants de la composition. Par ailleurs, cette température élevée complique fortement la fabrication des produits cosmétiques (outillage spécifique), augmentant leurs prix de revient, En outre, avec cette température de fusion élevée, le composé se présente sur la peau, après application, à l'état cristallin ; cet état trop rigide ne peut pas remplir les espaces intercornéocytaires de la peau.
Aussi, la demanderesse a cherché un moyen pour abaisser le point de fusion des céramides de type III sous forme d'un seul stéréoisomère.

De façon surprenante, elle a trouvé que l'association d'un céramide de type V de synthèse à un céramide de type III sous forme d'un seul stéréoisomére permettait la formation d'un mélange dont la température de fusion est inférieure à celle du céramide de type III sous forme d'un seul stéréoisomère.

L'invention a donc pour objet une composition cosmétique ou dermatologique contenant un mélange d'au moins un céramide de type III sous forme d'un seul stéréoisomère et d'au moins un céramide de type V de synthèse.

L'invention a encore pour objet un procédé d'abaissement du point de fusion d'au moins un céramide de type III sous forme d'un seul stéréoisomère, consistant à mélanger à ce céramide au moins un céramide de type V de synthèse.

Le céramide de type III sous forme d'un seul stéréoisomère est avantageusement un céramide obtenu par voie enzymatique.

En particulier, le mélange de céramides est un mélange d'au moins un céramide de type III sous forme d'un seul stéréoisomère, d'au moins un céramide de type V de synthèse et d'au moins un céramide de type Il de synthèse. II est aussi possible d'utiliser un mélange de céramides d'au moins un céramide de type Ill sous forme d'un seul stéréoisomère, d'au moins un céramide de type V de synthèse, d'au moins un céramide de type Il de synthèse et/ou d'au moins un céramide de type IV de synthèse.

De façon générale les céramides auxquels s'appliquent l'invention répondent à la formule (I) suivante : 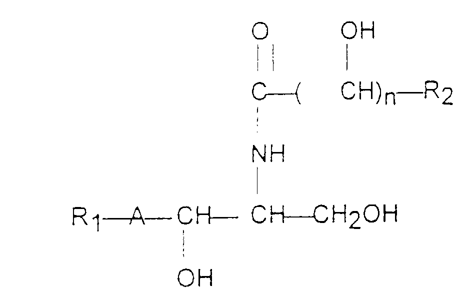 où A représente -CH₂-, 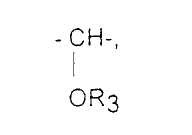 ou -CH=CH- ;
R₁ représente une chaîne alkyle linéaire ou ramifiée, saturée ou insaturée, en C₁₀ à C₂₆ ;
R₂ représente une chaîne alkyle linéaire ou ramifiée, saturée ou insaturée, en C₁₂ à C₃₆; R₃ représente H ou -CO-CHOH-R₂;
n représente 0 ou 1.

Comme céramide de type III utilisable dans l'invention, on peut citer la phytosphingosine acylée par un acide gras ayant de 12 à 30 atomes de carbone et notamment par l'acide palmitique, l'acide tétracosanéïque, l'acide linoléique ou l'acide stéarique. En particulier, on peut utiliser le céramide III vendu par la société Brocades qui est le N-stéaroyl-phytosphingosine ou encore le N-{2,3-dihydroxy-(1-hydroxyméthyl)-heptadécyl}-octadécanamide. Ce produit est optiquement actif (dextrogyre).

Comme céramide de type V de synthèse utilisable dans l'invention, on peut citer la sphingosine acylée par un acide gras hydroxylé ayant de 12 à 20 atomes de carbone et notamment par l'acide 2-hydroxystéarique ou 2-hydroxypalmitique. En particulier, on utilise la N-α-hydroxypalmitoyldihydrosphingosine connu aussi sous le nom 2-(2'-hydroxy hexadécanoyl)amino octadécane-1,3-diol.

Le céramide Il de synthèse est par exemple une sphinganine N-acylée par un acide gras ayant de 12 à 20 atomes de carbone comme l'acide oléïque, linoléïque, laurique, myristique, telle que la N-oléoyl-dihydrosphingosine et le céramide de type IV de synthèse est notamment une sphinganine N-acylée par un hydroxyacide de 21 à 36 atomes de carbone comme la N-α-hydroxybéhénoyl-dihydrosphingosine appelé encore le 2-(2-hydroxydocosanoyl)amino octadécane-1,3-diol.

De préférence, le céramide de type V de synthèse et éventuellement le céramide de type II et/ou IV sont présents en une quantité suffisante pour abaisser le point de fusion du mélange à une température inférieure à 90 °C et mieux inférieure à 85 °C.

En traçant le diagramme de phases, il est possible pour l'homme du métier de déterminer la quantité de chaque céramide et notamment de céramide V de synthèse permettant d'obtenir un point de fusion du mélange inférieur à 90 °C et mieux inférieur à 85 °C.

L'abaissement de la température de fusion conduit à un produit amorphe ou cristal liquide, après application sur la peau, plus efficace, notamment vis-à-vis de l'effet barrière, qu'un produit cristallin.

Pour un mélange de céramides III et V, on peut utiliser 65 % en poids de céramide V pour 35 % en poids de céramide III, par rapport au poids total de la composition, ce qui permet d'abaisser le point de fusion de 126 °C à 82,7 °C.

Pour le mélange de céramides III, V et Il, ou peut utiliser 10 % en poids de céramide III, 50 % en poids de céramide V et 40 % en poids de céramide Il, par rapport au poids total de la composition, ce qui permet d'abaisser le point de fusion de 126 °C à 77,4 °C ou utiliser 20 % de céramide III, 40 % en poids de céramide V et 40 % en poids de céramide Il, par rapport au poids total de la composition, ce qui permet d'abaisser le point de fusion de 126 °C à 69,8 °C.

Il est aussi possible d'utiliser un mélange de céramides III, V, II et IV pour abaisser la température de fusion jusqu'à 77,8 °C, avec respectivement, en % en poids par rapport au poids total de la composition, 8 % de céramide III, 40 % de céramide V, 32 % de céramide Il et 20 % de céramide IV.

Cette composition est bien adaptée à l'hydratation de la peau. Aussi, l'invention a encore pour objet l'utilisation de la composition cosmétique décrite ci-dessus pour hydrater la peau. Toutefois, la composition de l'invention peut aussi être utilisée dans le traitement des xéroses et dans tout traitement de la peau où il est nécessaire de protéger la peau. En effet, les céramides sont connus pour leur effet barrière.

L'invention a aussi pour objet l'utilisation de la composition définie précédemment pour préparer une pommade ou un onguent destiné au traitement thérapeutique des peaux sèches.

L'invention a enfin pour objet un procédé de traitement cosmétique de la peau, caractérisé en ce qu'il consiste à appliquer sur la peau une composition telle que définie ci-dessus.

Les compositions de l'invention peuvent en outre contenir tous les constituants classiquement utilisés dans les compositions cosmétiques ou dermatologiques. En particulier elles peuvent contenir une huile végétale (tournesol, germe de maïs), minérale (vaseline), siliconée (cyclométhicone), fluorée (perfluoropolyéther) ou synthétique (huile de purcellin, myristate d'isopropyle, octanoate de cétéaryle, monostéarate de glycérol), une phase aqueuse, des adjuvants hydrophiles comme les gélifiants, les antioxydants (vitamine E), les conservateurs, les opacifiants, les neutralisants, les complexants, des adjuvants lipophiles tels que les huiles essentielles, les colorants, les alcools gras, les acides gras, les cires, les parfums, ainsi que les pigments (oxydes de titane ou de zinc) et les charges. Ces adjuvants peuvent représenter, au total, de 0,1 % à 20 % du poids total de la composition.

La composition de l'invention peut se présenter sous toutes les formes galéniques classiquement utilisées dans les domaines considérés. Ainsi, la composition selon l'invention peut se présenter sous forme d'une solution aqueuse ou huileuse, d'un gel aqueux, d'une émulsion eau-dans-huile (E/H) ou huile-dans-eau (H/E), d'une émulsion triple eau/huile/eau ou d'une dispersion de vésicules lipidiques (ioniques ou non ioniques). Cette composition peut avoir l'aspect d'une crème, d'un sérum, d'une lotion ou d'un lait.

Pour une émulsion, on utilise, selon le cas, un système émulsionnant (E/H) ou (H/E). Lors de l'utilisation d'une dispersion de vésicules lipidiques, ces dernières peuvent constituer le système émulsionnant. La quantité de système émulsionnant est choisie classiquement de 0,1% à 10% du poids total de la composition.

Comme émulsionnant H/E utilisable dans l'invention, on peut citer le PEG-50 stéarate et le PEG-40 stéarate vendus respectivement sous les dénominations commerciales MYRJ 53 et MYRJ 52 par la Société ICI, le sorbitan tristéarate vendu sous la dénomination commerciale SPAN 65 par la Société ICI et le sorbitan stéarate vendu sous la dénomination commerciale SPAN 60 par la Société ICI.

Comme émulsionnant (E/H) utilisable dans l'invention, on peut citer le mélange polyglycéryl-4 isostéarate/cétyldiméthicone copolyol/hexyl laurate vendu sous la dénomination commerciale ABIL WE 09 par la Société GOLDSCHMIDT et l'isostéaryl diglycéryl succinate vendu sous la dénomination commerciale IMWITOR 780 K par la Société HÜLS ou encore des sucres.

Lorsque la composition a la forme d'un gel, on utilise les gélifiants classiques comme les polysaccharides (gomme de xanthane, de caroube) et les polymères carboxyvinyliques.

Les compositions de l'invention peuvent, de plus contenir d'autres actifs que les céramides. Ces actifs peuvent être des actifs hydrophiles comme des agents hydratants tels que l'urée, les protéines et leurs hydrolysats (acides aminés notamment), les polyols (glycérine, sorbitol), des agents cicatrisants comme l'allantoïne ainsi que ses dérivés. Ces actifs peuvent aussi être des actifs lipophiles comme les vitamines (vitamines A, F, B) et leurs dérivés. Ces actifs supplémentaires peuvent aussi être des filtres hydrophiles ou lipophiles pour filtrer les rayons visibles et/ou ultraviolets comme l'octyl méthoxycinnamate ou encore des actifs dermatologiques. Ces actifs peuvent représenter, au total, de 0,1 % à 10 % du poids total de la composition.

La description qui suit est donnée à titre illustratif et non limitatif. Les pourcentages des exemples sont donnés en poids.

### Exemple 1 : Emulsion huile-dans-eau hydratante

| | |
|---|---|
| - Silicone volatile 7158 de chez Union Carbide | 10,0 |
| - Perhydrosqualène | 18,0 |
| - Huile de vaseline | 5,0 |
| - Lanoline liquide | 4,0 |
| - Arlacel 165 de chez Atlas | 6,0 |
| - Tween 60 de chez Atlas | 2,0 |
| - N-stéaroyl-phytosphingosine | 0,52 |
| - N-α-hydroxypalmitoyl-dihydrosphingosine | 0,36 |
| - Alcool cétylique | 1,2 |
| - Alcool stéarique | 2,5 |
| - Hydroxyde de sodium | 0,008 |
| - Propylène glycol | 5,0 |
| - Triéthanolamine | 0,1 |
| - Conservateur | 0,3 |
| - Anti-oxydant | 0,3 |
| - Eau déminéralisée qsp | 100 |

L'émulsion se présente sous la forme d'une crème blanche à appliquer le soir pour réparer et hydrater la peau. Elle est destinée à toutes les peaux.

### Exemple 2 : Emulsion huile-dans-eau hydratante

| | |
|---|---|
| - Huile de germe de maïs | 2,0 |
| - Monostéarate de glycérol | 3,0 |
| - PEG 400 | 3,0 |
| - Carbopol 941 | 0,2 |
| - Myristate d'isopropyle | 1,0 |
| - N-stéaroyl-phytosphingosine | 0,2 |
| - N-α-hydroxypalmitoyl-dihydrosphingosine | 0,1 |
| - Alcool cétylique | 3,0 |
| - Alcool stéarique | 3,0 |
| - Hydroxyde de sodium | 0,008 |
| - Propylène glycol | 5,0 |
| - Conservateur | 0,3 |
| - Parfum | 0,5 |
| - Eau déminéralisée qsp | 100 |

Cette émulsion est une crème blanche de jour, hydratante, utilisable pour tous types peaux.

### Exemple 3: Emulsion eau-dans-huile hydratante

| | |
|---|---|
| - Huile de vaseline | 10,0 |
| - Protegin X de chez Goldschmidt | 20,0 |
| - Huile de tournesol | 15,0 |
| - Composition aromatique | 1,0 |
| - N-stéaroyl-phytosphingosine | 0,02 |
| - N-α-hydroxypalmioyl-dihydrosphingosine | 0,04 |
| - N-oléoyl-dihydrosphingosine | 0,04 |
| - Sulfate de magnésium | 0,5 |
| - Glycérol | 5,0 |
| - Cétiol HE de chez Henkel | 4,0 |
| - Conservateur | 0,3 |
| - Eau déminéralisée qsp | 100 |

Cette crème est plus spécialement destinée au traitement de nuit des peaux sèches sensibles.

### Exemple 4: Emulsion eau-dans-huile hydratante

| | |
|---|---|
| - Abil We 09 de chez Goldschmidt 5,0 | |
| - Myristate d'isopropyle | 5,0 |
| - Silicone volatile 7158 d'Union Carbide | 8,0 |
| - Aérosil R 812 de chez Dégussa | 0,4 |
| - Huile de Purcellin de chez Dragocco | 14,0 |
| - Chlorure de sodium | 0,5 |
| - Transcutol de chez Gattefosse | 3,0 |
| - N-α-hydroxybéhénoyl-dihydrosphingosine | 1,0 |
| - N-stéaroyl-phytosphingosine | 0,04 |
| - N-α-hydroxypalmitoyl-dihydrosphingosine | 0,25 |
| - N-oléoyl-dihydrosphingosine | 1,6 |
| - Hydroxyde de sodium | 0,008 |
| - Huile de vaseline | 5,0 |
| - Conservateur | 0,3 |
| - Eau déminéralisée qsp | 100 |

Cette crème est plus spécialement destinée au traitement de nuit des peaux sèches.

### Exemple 5 : Gel hydroalcoolique

| | |
|---|---|
| - Carbopol 940 | 0,9 |
| - N-stéaroyl-phytosphingosine | 0,2 |
| - N-α-hydroxypalmitoyl-dihydrosphingosine | 0,1 |
| - Alcool éthylique | 20,0 |
| - Triéthanolamine | 0,3 |
| - Propylène glycol | 5,0 |
| - Transcutol | 5,0 |
| - Conservateur | 0,3 |
| - Parfum | 0,3 |
| - Eau déminéralisée qsp | 100 |

Ce gel est destiné à l'hydratation et restructuration des peaux sèches.

### Exemple 6 : Gel émulsionné huile-dans-eau

| | |
|---|---|
| - Carbopol 940 | 0,6 |
| - Silicone volatile 7158 d'Union Carbide | 3,0 |
| - Huile de Purcellin de chez Dragocco | 7,0 |
| - N-stéaroyl-phytosphingosine | 0,06 |
| - N-α-hydroxypalmitoyl-dihydrosphingosine | 0,04 |
| - Alcool éthylique | 10,0 |
| - Triéthanolamine | 0,2 |
| - Tefosse 63 de chez Gattefosse | 3,0 |
| - Cétiol HE | 2,0 |
| - Caféïne | 1,0 |
| - Conservateur | 0,3 |
| - Parfum | 0,4 |
| - Eau déminéralisée qsp | 100 |

Ce gel est destiné à l'hydratation du corps de tout type de peau.

### Exemple 7 : Gel aqueux

| | |
|---|---|
| - Carbopol 940 | 0,6 |
| - Transcutol | 5,0 |
| - Triéthanolamine | 0,3 |
| - Conservateur | 0,3 |
| - Propylène glycol | 3,0 |
| - Hydroxyde de sodium | 0,007 |
| - N-α-hydroxybéhénoyl-dihydrosphingosine | 0,03 |
| - N-stéaroyl-phytosphingosine | 0,05 |
| - N-α-hydroxypalmitoyl-dihydrosphingosine | 0,02 |

Ce gel est plutôt destiné à l'hydratation du corps des peaux sensibles.

### Exemple 8 : Crème aux liposomes non ioniques

| | |
|---|---|
| - Carbopol 940 | 0,2 |
| - Transcutol | 3,0 |
| - Triéthanolamine | 0,2 |
| - Conservateur | 0,3 |
| - Polyglycéryl-3-cétyl éther | 3,8 |
| - B-sitostérol | 3,8 |
| - Dicétyl-phosphate | 0,4 |
| - Hydroxyde de sodium | 0,007 |
| - N-oléyl-dihydrosphingosine | 0,2 |
| - N-stéaroyl-phytosphingosine | 0,1 |
| - Huile de tounesol | 35,0 |
| - N-α-hydroxypalmitoyl-dihydrosphingosine | 0,25 |
| - Parfum | 0,6 |
| - Eau déminéralisée qsp | 100 |

Ce gel est plutôt destiné à l'hydratation et la restructuration des peaux sèches, notamment pendant la nuit.

Les mélanges de céramides donnés dans les exemples sont tous des mélanges présentant un point de fusion plus bas que ceux des céramides pris isolément entrant dans le mélange.

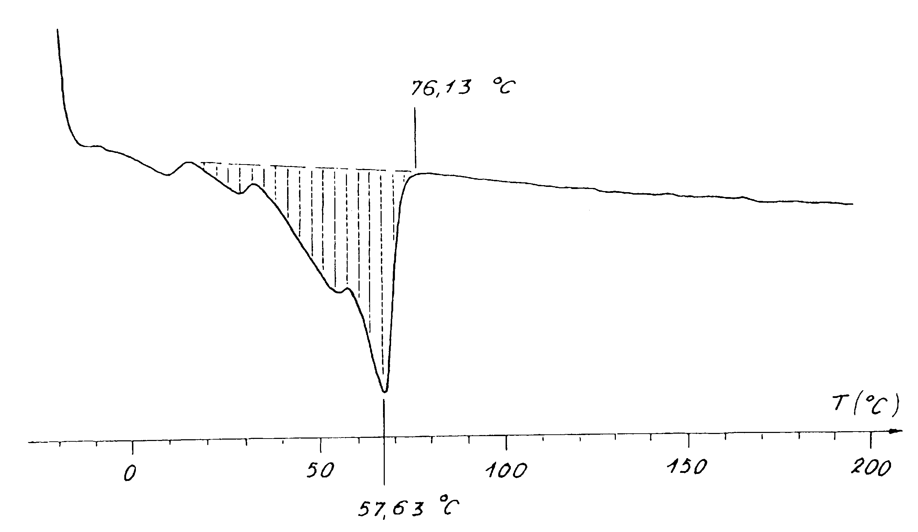

A titre d'exemple, la figure annexée donne, pour le mélange 20 % en poids de céramide de type III, 40 % en poids de céramide de type V et 40 % en poids de céramide de type Il, une courbe de calométrie à balayage différentielle (DSC). Cette courbe donne la différence de température entre une cellule de référence (vide) et une cellule de mesure contenant le mélange de céramides en fonction de la température du four contenant ces cellules de mesure et de référence, donnée en°C. La montée en température est effectuée à raison de 10 °C/min. Le pic de fusion du mélange correspond à une température de 67,63 °C.

## Revendications

1. Composition cosmétique ou dermatologique contenant un mélange d'au moins un céramide de type III sous forme d'un seul stéréoisomère et d'au moins un céramide de type V.

2. Composition selon la revendication 1, caractérisée en ce que le mélange est un mélange d'au moins un céramide de type III, d'au moins un céramide de type V et d'au moins un céramide de type II.

3. Composition selon l'une des revendications précédentes, caractérisée en ce que les céramides différent du type III sont présents en une quantité suffisante pour abaisser le point de fusion du mélange à une température inférieure à 90 °C.

4. Composition selon l'une des revendications précédentes, caractérisée en ce que les céramides répondent à la formule (I) suivante : 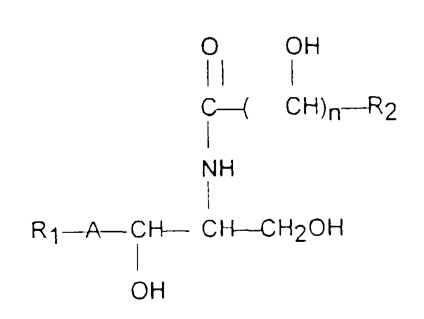 où A représente 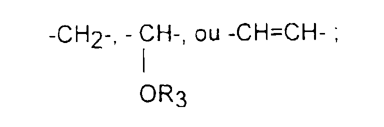
R₁ représente une chaîne alkyle linéaire ou ramifiée, saturée ou insaturée, en C₁₀ à C₂₆ ;
R₂ représente une chaîne alkyle linéaire ou ramifiée, saturée ou insaturée, en C₁₂ à C₃₆ ;
R₃ représente H ou -CO-CHOH-R₂ ;
n représente 0 ou 1.

5. Composition selon l'une des revendications précédentes, caractérisée en ce que le mélange de céramide contient 20 % en poids de céramide de type III, 40 % en poids de céramide de type V et 40 % en poids de céramide de type Il, par rapport au poids total de la composition.

6. Composition selon l'une des revendications 2 à 5, caractérisée en ce que le céramide de type II est la N-oléoyidihydrosphingosine.

7. Composition selon l'une des revendications précédentes, caractérisée en ce que le céramide de type V est la N-α-hydroxypalmitoyl-dihydrosphingosine.

8. Composition selon l'une des revendications précédentes, caractérisée en ce que le céramide de type III est la N-stéaroyl phytosphingosine.

9. Utilisation de la composition selon l'une des revendications précédentes pour hydrater la peau.

10. Utilisation de la composition selon l'une quelconque des revendications 1 à 8 pour préparer une pommade ou un onguent destiné au traitement thérapeutique des peaux sèches.

11. Procédé de traitement cosmétique de la peau. caractérisé en ce qu'il consiste à appliquer sur la peau une composition selon l'une quelconque des revendications 1 à 8.

12. Procédé pour abaisser le point de fusion d'au moins un céramide de type II sous forme d'un seul stéréoisomère, consistant à mélanger à ce céramide au moins un céramide de type V.

13. Procédé selon la revendication 12. caractérisé en ce que les céramides différents du type II sont présents en une quantité suffisante pour abaisser le point de fusion du mélange à une température inférieure à 90 °C et mieux inférieure à 85 °C.

## Claims

1. Cosmetic or dermatological composition containing a mixture of at least one type-III ceramide in the form of a single stereoisomer and of at least one type-V ceramide of synthesis.

2. Composition according to Claim 1, characterized in that the mixture is a mixture of at least one type-III ceramide, of at least one type-V ceramide of synthesis and of at least one type-II ceramide of synthesis.

3. Composition according to one of the preceding claims, characterized in that the ceramides, other than type-III ceramides, are present in an amount which is sufficient to lower the melting point of the mixture to a temperature below 90°C.

4. Composition according to one of the preceding claims, characterized in that the ceramides correspond to the following formula (I): 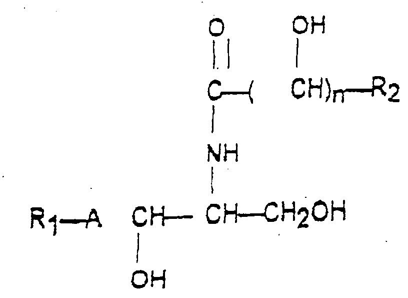 where A represents -CH₂-, 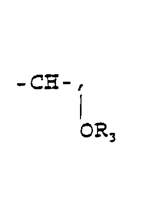 or -CH=CH-;
R₁ represents a saturated or unsaturated, linear or branched, C₁₀ to C₂₆ alkyl chain;
R₂ represents a saturated or unsaturated, linear or branched, C₁₂ to C₃₆ alkyl chain;
R₃ represents H or -CO-CHOH-R₂;
n represents 0 or 1.

5. Composition according to one of the preceding claims, characterized in that the ceramide mixture contains 20 % by weight of type-III ceramide, 40 % by weight of type-V ceramide and 40 % by weight of type-II ceramide, with respect to the total weight of the composition.

6. Composition according to one of Claims 2 to 5, characterized in that the type-II ceramide is N-oleoyldihydrosphingosine.

7. Composition according to one of the preceding claims, characterized in that the type-V ceramide is N-(α-hydroxypalmitoyl)dihydrosphingosine.

8. Composition according to one of the preceding claims, characterized in that the type-III ceramide is N-stearoylphytosphingosine.

9. Use of the cosmetic composition according to one of the preceding claims for moisturizing the skin, methods for treatment of the human body by therapy being excluded.

10. Use of the composition according to any one of Claims 1 to 8 for preparing a salve or an ointment intended for the therapeutic treatment of dry skins.

11. Process for the cosmetic treatment of the skin, characterized in that it consists in applying a composition according to any one of Claims 1 to 8 to the skin.

12. Process for lowering the melting point of at least one type-III ceramide in the form of a single stereoisomer which consists in mixing at least one type-V ceramide with this ceramide.

13. Process according to Claim 12, characterized in that the ceramides, other than type-III ceramides, are present in an amount which is sufficient to lower the melting point of the mixture to a temperature of less than 90°C and better less than 85°C.

## Patentansprüche

1. Kosmetische oder dermatologische Zusammensetzung, die ein Gemisch aus mindestens einem Ceramid vom Typ III in Form eines einzigen Stereoisomers und mindestens einem Synthetischen Ceramid vom Typ V enthält.

2. Zusammensetzung nach Anspruch 1, dadurch gekennzeichnet, daß das Gemisch ein Gemisch aus mindestens einem Ceramid vom Typ III, mindestens einem Synthetischen Ceramid vom Typ V und mindestens einem synthetischen Ceramid vom Typ II ist.

3. Zusammensetzung nach einem der vorhergehenden Ansprüche, dadurch gekennzeichnet, daß die verschiedenen Ceramide vom Typ III in einer Menge enthalten sind, die für die Absenkung des Schmelzpunkts des Gemischs auf eine Temperatur unter 90°C ausreichend ist.

4. Zusammensetzung nach einem der vorhergehenden Ansprüche, dadurch gekennzeichnet, daß die Ceramide die folgende allgemeine Formel (I) 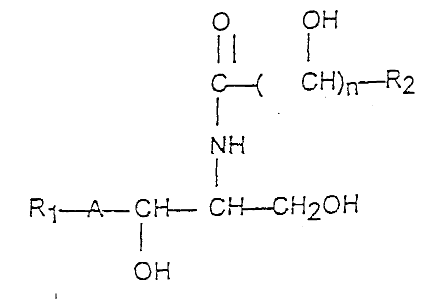 aufweisen, worin bedeuten: 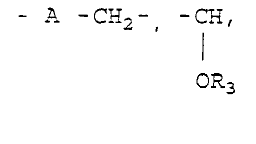 oder -CH=CH-,
- R₁ eine geradkettige oder verzweigte, gesättigte oder ungesättigte Alkylkette mit 10 bis 26 Kohlenstoffatomen,
- R₂ eine geradkettige oder verzweigte, gesättigte oder ungesättigte Alkylkette mit 12 bis 36 Kohlenstoffatomen,
- R₃ Wasserstoff oder -CO-CHOH-R₂ und
- n die Zahl 0 oder 1.

5. Zusammensetzung nach einem der vorhergehenden Ansprüche, dadurch gekennzeichnet, daß das Ceramidgemisch 20 Gew.-% Ceramid vom Typ III, 40 Gew.-% Ceramid vom Typ V und 40 Gew.-% Ceramid vom Typ II, bezogen auf das Gesamtgewicht der Zusammensetzung, enthält.

6. Zusammensetzung nach einem der Ansprüche 2 bis 5, dadurch gekennzeichnet, daß das Ceramid vom Typ II N-Oleoyldihydrosphingosin ist.

7. Zusammensetzung nach einem der vorhergehenden Ansprüche, dadurch gekennzeichnet, daß es sich bei dem Ceramid vom Typ V um N-α-Hydroxypalmitoyldihydrosphingosin handelt.

8. Zusammensetzung nach einem der vorhergehenden Ansprüche, dadurch gekennzeichnet, daß es sich bei dem Ceramid vom Typ III um N-Stearoylphytosphingosin handelt.

9. Verwendung der kosmetischen Zusammensetzung nach einem der vorhergehenden Ansprüche für die Hydratisierung der Haut, unter Ausschluß der Verfahren zur therapeutischen Behandlung des menschlichen Körpers.

10. Verwendung der Zusammensetzung nach einem der Ansprüche 1 bis 8 für die Herstellung einer Pomade oder einer Salbe, die für die therapeutische Behandlung von trokkener Haut bestimmt ist.

11. Verfahren zur kosmetischen Behandlung der Haut, dadurch gekennzeichnet, daß auf die Haut eine Zusammensetzung nach einem der Ansprüche 1 bis 8 aufgetragen wird.

12. Verfahren zur Absenkung des Schmelzpunktes von mindestens einem Ceramid vom Typ III in Form eines einzigen Stereoisomers, das darin besteht, dieses Ceramid mit mindestens einem Ceramid vom Typ V zu vermischen.

13. Verfahren nach Anspruch 12, dadurch gekennzeichnet, daß die verschiedenen Ceramide vom Typ III in einer Menge enthalten sind, die für die Absenkung des Schmelzpunkts des Gemischs auf eine Temperatur unter 90 °C und besser unter 85°C ausreichend ist.
